Europäisches Patentamt

European Patent Office

Office européen des brevets

(19)

(11) Veröffentlichungsnummer: **0 054 781**
**B1**

(12) ## EUROPÄISCHE PATENTSCHRIFT

(45) Veröffentlichungstag der Patentschrift:
10.10.84

(51) Int. Cl.³: **A 61 N 1/04**

(21) Anmeldenummer: **81110126.0**

(22) Anmeldetag: **04.12.81**

(54) **Implantierbare Elektrode.**

(30) Priorität: **23.12.80 CH 9523/80**

(43) Veröffentlichungstag der Anmeldung:
**30.06.82 Patentblatt 82/26**

(45) Bekanntmachung des Hinweises auf die Patenterteilung:
**10.10.84 Patentblatt 84/41**

(84) Benannte Vertragsstaaten:
**CH DE FR GB IT LI NL SE**

(56) Entgegenhaltungen:
**EP - A - 0 042 551**
**FR - A - 2 225 179**
**US - A - 535 466**
**US - A - 3 749 101**
**US - A - 4 010 759**
**US - A - 4 011 861**
**US - A - 4 030 508**
**US - A - 4 156 429**

**MEDICAL AND BIOLOGICAL ENGINEERING, Band 11, Nr. 5, September 1973, Seiten 659, 660 Stevenage, G.B. W. MINDT et al.: "Stimulating electrode with low energy consumption"**

(73) Patentinhaber: **KONTRON AG, Bernerstrasse 169 Süd, CH-8048 Zürich (CH)**
Patentinhaber: **W.C. Heraeus GmbH, Heraeusstrasse 12 - 14, D-6450 Hanau / Main (DE)**

(72) Erfinder: **Bussard, Adrien, Stöcklen, CH-6344 Meierskappel (CH)**
Erfinder: **Aldinger, Fritz, Barbarossastrasse 44, D-6458 Rodenbach 2 (DE)**
Erfinder: **Sperner, Franz, Friedenstrasse 59, D-6450 Hanau (DE)**

(74) Vertreter: **Buntz, Gerhard et al, Grenzacherstrasse 124 Postfach 3255, CH-4002 Basel (CH)**

**Beschreibung**

Die Erfindung bezieht sich auf eine poröse Elektrode zur Implantation in organisches Gewebe, insbesondere eine Herzschrittmacher-Elektrode.

Aus der US-Patentschrift 4 011 861 sind poröse Elektroden zur Implantation in organisches Gewebe, insbesondere Herzschrittmacher-Elektroden, bekannt. Sie bestehen aus einem elektrisch leitfähigen Teil, dessen Oberfläche mit Körperflüssigkeit in Kontakt treten kann und von einem inerten porösen Material vollständig umhüllt ist, dessen Poren einen Durchmesser von 0,5 bis 1000 µm besitzen. Der elektrisch leitfähige Teil kann kompakt oder auch porös sein. Als Werkstoff für diesen Teil sind Platin, Iridium, Niob, Indium, Palladium, Titan, Tantal, Vanadium, Wolfram, Chrom, Kobalt, rostfreier Stahl, Legierungen von einigen dieser Metalle sowie Kohlenstoff genannt. Die poröse Hüllschicht kann durch Sintern hergestellt und elektrisch leitend oder elektrisch nicht leitend sein. Wenn diese Hüllschicht elektrisch leitend ist, so kann sie ebenfalls aus Metall, einer Metall-Legierung oder Metallverbindung eines Elements aus der Gruppe Tantal, Titan, Niob oder aus einer Legierung auf Kobalt-Chrom-Basis oder aus Kohlenstoff, einem mit Metall überzogenen Nichtleiter oder einem elektrisch leitenden Kunststoff bestehen. Ist diese Hüllschicht elektrisch nicht leitend, so kann sie aus Keramik, Aluminiumoxid, Siliziumdioxid, Kalzium-, Magnesium-, Titanund/oder Zirkoniumoxid oder einer unter dem Handelsnamen Cerosium erhältlichen porösen Keramik aus solchen Oxiden bestehen.

Aus der US-Patentschrift 3 476 116 ist eine rohrförmige Elektrode für Herzschrittmacher bekannt, die aus einem zweistufigen Gehäuse aus dielektrischem Werkstoff, wie Kunststoff, besteht, das eine Spirale aus Platin-Iridium enthält, deren Durchmesser dem Durchmesser des Rohres der jeweiligen Gehäusestufe angepaßt ist. Der Boden des Gehäuses ist mit einer Öffnung von 1 mm$^2$ versehen. Das Gehäuse ist mit einer elektrisch leitfähigen Lösung, wie wäßriger Salzlösung, gefüllt.

Der Erfindung liegt die Aufgabe zugrunde, eine in organisches Gewebe implantierbare Elektrode zu schaffen, die eine niedrige Reizschwelle besitzt, die chronische Reizschwelle außerordentlich schnell erreicht, darüber hinaus eine minimale Überhöhung der Reizschwelle zwischen intraoperativem und maximalem, vorübergehenden Reizschwellenwert aufweist und eine möglichst niedrige Stimulationsleistung erfordert.

Gelöst wird diese Aufgabe durch eine Elektrode der eingangs charakterisierten Art erfindungsgemäß dadurch, daß sie aus einem Sinterkörper aus elektrisch leitenden Teilchen besteht, die aus einem Metall, einer Metall-Legierung oder Metallverbindung eines Elements aus der Gruppe Tantal, Titan, Niob und Zirkonium oder aus einer Legierung auf Kobalt-Chrom-Basis bestehen, wobei wenigstens in einem Teil der Oberfläche der Elektrode die diesen Oberflächenbereich bildenden Teilchen an den nicht miteinander verbundenen Stellen mit einem elektrisch gut leitenden, gegenüber Körperflüssigkeit inerten Metall versehen sind und außerhalb des genannten Oberflächenbereichs der Elektrode die Teilchen — wenigstens in einem an die Oberfläche grenzenden Bereich — an den nicht miteinander verbundenen Stellen mit einer dünnen Schicht aus einem Werkstoff mit geringerer elektrischer Leitfähigkeit als der Werkstoff der Teilchen versehen sind.

Als Metall, das gegenüber der Körperflüssigkeit inert ist und mit dem ein Teil der Oberfläche des Sinterkörpers versehen ist, hat sich insbesondere Platin, Iridium oder Platin-Iridium-Legierungen mit einem Iridium-Gehalt bis zu 20 Gew.-%, Rest Platin, bewährt.

Gute Ergebnisse wurden mit Elektroden erzielt, bei denen die Teilchen aus Tantal bestehen. Insbesondere hat sich ein Sinterkörper aus anodisiertem Tantal bewährt.

Vorteilhafterweise wird die Oberfläche des Sinterkörpers teilweise mit Platin versehen, insbesondere, wenn Tantal als Teilchenwerkstoff oder anodisiertes Tantal verwendet werden.

Die Erfindung ist jedoch nicht auf die vorgenannte Werkstoff-Auswahl und Werkstoff-Kombination beschränkt.

Als Teilchenwerkstoff kann beispielsweise auch elektrisch leitfähiges $TiO_x$ (x = 0,25 bis 1,5) verwendet werden. Durch Oxidation werden dann im Oberflächenbereich der Elektrode die Teilchen an den Stellen, an denen sie nicht miteinander versintert sind, mit einer dünnen elektrisch nicht leitenden $TiO_2$-Schicht versehen.

Ebenfalls geeignet als Teilchenwerkstoffe sind beispielsweise zu nennen: Nitride, Carbide oder Carbonitride von Ta, Ti, Nb oder Zr; Ta-W-Legierungen mit bis zu 10 Gew.-% W; Ti-Al-Fe-Legierungen mit bis zu 6 Gew.-% Al und mit bis zu 2,5 Gew.-% Fe.

Bei Elektroden gemäß der Erfindung ist der größte Teil der Poren zum Körpergewebe hin offen und hat die Porengröße vorteilhafterweise einen Durchmesser im Bereich von 10 bis 50 µm.

Erfindungsgemäße Elektroden besitzen überraschenderweise eine Reizschwellenhöhe von etwa 0,3 V chronisch, die mit der intraoperativen vergleichbar ist. Bei bekannten Herzschrittmacherelektroden wurde unter vergleichbaren Bedingungen eine Reizschwellenhöhe von 2 bis 3 V chronisch und 0,4 bis 1 V intraoperativ gemessen. Die chronische Reizschwellenhöhe ist also bei erfindungsgemäßen Elektroden etwa eine Zehnerpotenz kleiner. Ebenso günstige Meßergebnisse konnten für die Stimulationsenergie verifiziert werden. Sie beträgt für erfindungsgemäße Elektroden etwa 0,15 bis 0,2 µJ, und ist damit etwa zwei Zehnerpotenzen kleiner als die üblichen Elektroden, deren Stimulationsenergie etwa 15 bis 20 µJ beträgt.

Die Stimulationsspannung liegt bei erfindungsgemäßen Elektroden unterhalb der Spannung, bei der sich die Körperflüssigkeit zersetzt.

Aufgrund dieser vorteilhaft niedrigen Werte besitzen Herzschrittmacher mit erfindungsgemäßen Elektroden eine wesentlich größere Lebensdauer; bei gleicher Batteriekapazität der Schrittmacher steigt die Lebensdauer wenigstens auf das Doppelte an. Herzschrittmacher mit erfindungsgemäßer Elektrode benötigen im Betrieb nur eine Batterie ohne Spannungsverdoppelung, während man bisher entweder zwei in Serie geschaltete Batterien oder eine Batterie mit Spannungsverdoppelung benötigte.

Diese erfindungsgemäßen Vorteile ergeben sich besonders deutlich bei solchen Elektroden, die becherförmig ausgebildet sind und bei denen der Becher aus einem porösen Sinterkörper aus wenigstens in seinem Oberflächenbereich anodisiertem Tantal besteht, dessen Innenwände und gegebenenfalls dessen Rand mit einer dünnen Platinschicht versehen sind, und der größte Teil der Poren des Sinterkörpers einen Durchmesser im Bereich von 10 bis 50 µm besitzt.

Aus den erwähnten Vorteilen erfindungsgemäßer Elektroden resultiert, daß bei ihrem Einsatz Gewebereizungen erheblich herabgesetzt sind. Die Elektroden besitzen innerhalb weniger Millisekunden nach erfolgtem Stimulationsimpuls wieder volle »Sensing«-Empfindlichkeit. Sie können leicht, beispielsweise durch Subclaviapunktierung mittels normaler Einführbestecke, also ohne übergroße Einführbestecke, eingeführt werden, so daß Gefäßverletzungen, wie sie bei Verwendung übergroßer Einführbestecke zu befürchten sind, weitgehend vermieden werden.

In den Figuren sind Ausführungsbeispiele erfindungsgemäßer Elektroden im Vertikalschnitt dargestellt. Es zeigt

Fig. 1 eine zylindrische Elektrode,

Fig. 2 eine Elektrode in Becherform,

Fig. 3 eine Elektrode in Becherform mit Durchgängen in der Becherwand.

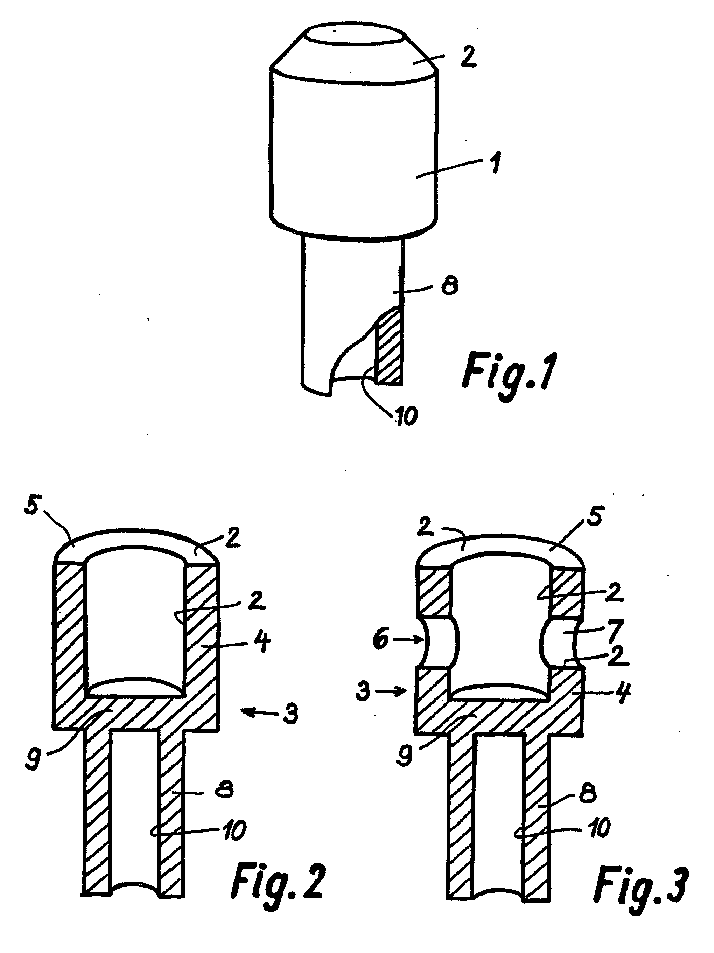

Die in Fig. 1 dargestellte Elektrode 1 besteht aus einem porösen Sinterkörper aus an den freien Oberflächen, d. h., an den nicht miteinander verbundenen Stellen der Tantalteilchen des Oberflächenbereichs der Elektrode anodisiertem Tantal. Im Bereich der Elektrodenspitze ist der Sinterkörper mit einer Platinschicht 2 versehen.

Die Herstellung dieser Elektrode erfolgt in der Weise, daß Tantalpulver mit einer Teilchengröße ≤ 60 µm, aber ≥ 40 µm in einer Form bei einem Druck von 2500 bar gepreßt und der gewonnene Preßling danach bei etwa 2200°C für eine Zeitdauer von etwa 1 bis 3 Stunden gesintert wird. Der Sinterkörper wird dann nach erfolgter Reinigung und Aktivierung in verdünnter Phosphorsäure anodisiert, so daß sich eine dünne Ta-Oxid-Schicht auf der freien Oberfläche der Ta-Teilchen ausbildet, d. h., an den nicht miteinander versinterten Stellen der Teilchen des Oberflächenbereichs der Elektrode. Zum Aufbringen der Platin-Schicht auf einen Teil der Sinterkörper-Oberfläche wird dieser Teil zur Beseitigung der Ta-Oxid-Schicht geschliffen und anschließend Platin aus einem handelsüblichen Platinbad galvanisch abgeschieden.

Fig. 2 zeigt eine becherförmige Elektrode. Der Becher besteht dabei aus einem porösen Sinterkörper 3 aus anodisiertem Tantal. Die Wand 4 und ggf. der Boden 9 sind innen mit Platinschichten 2 versehen. Es hat sich als vorteilhaft erwiesen, auch den Becherrand 5 mit einer Platinschicht 2 zu versehen.

Die Herstellung der becherförmigen Elektrode erfolgt in der Weise, daß aus Ta-Pulver mit einer Teilchengröße ≤ 60 µm, aber ≥ 40 µm ein becherförmiger Preßling hergestellt wird, wobei der Preßdruck 2500 bar beträgt. Dieser Preßling wird bei 2200°C für eine Zeitdauer von 1 bis 3 Stunden gesintert und der Sinterkörper nach erfolgter Reinigung und Aktivierung anschließend in verdünnter Phosphorsäure anodisiert. Nach Schleifen der Becherinnenwand und gegebenenfalls des Becherrandes wird auf den abgeschliffenen Oberflächen Platin aus einem handelsüblichen Platinbad galvanisch abgeschieden.

Die in Fig. 3 dargestellte Elektrode unterscheidet sich im wesentlichen von der gemäß Fig. 2 dadurch, daß die Wand 4 mit Durchgängen 6 versehen ist. Die Durchgangswände 7 können ebenfalls mit einer Platinschicht 2 versehen sein. Die Durchgänge werden mittels Hartmetallbohrern in die Wand eingebracht. Im übrigen erfolgt die Herstellung dieser becherförmigen Elektrode in gleicher Weise wie schon bei Fig. 2 beschrieben. Die Durchgänge werden in die Wand des Sinterkörpers gebohrt, bevor er anodisiert wird.

Die elektrische Zuführung erfolgt in allen dargestellten Ausführungsbeispielen über den Anschlußstutzen 8 an der Rückseite, wobei die Innenfläche des Stutzens 8, der aus anodisiertem Tantal besteht, entweder nur abgeschliffen oder nach Abschleifen noch galvanisch mit der Platin-Schicht 10 belegt ist.

**Patentansprüche**

1. Poröse Elektrode (1) zur Implantation in organisches Gewebe, insbesondere Herzschrittmacher-Elektrode, aus einem Sinterkörper (3) aus elektrisch leitenden Teilchen, die aus einem Metall, einer Metall-Legierung oder Metallverbindung eines Elements aus der Gruppe Tantal, Titan, Niob und Zirkonium oder aus einer Legierung auf Kobalt-Chrom-Basis bestehen, wobei wenigstens in einem Teil der Oberfläche der Elektrode (1) die diesen Oberflächenbereich bildenden Teilchen an den nicht miteinander verbundenen Stellen mit einem elektrisch gut leitenden, gegenüber Körperflüssigkeit inerten Metall versehen sind und außerhalb des genannten Oberflächenbereichs der Elektrode (1) die Teilchen — wenigstens in einem an die Oberfläche grenzenden Bereich — an den nicht miteinander verbundenen Stellen mit einer dünnen Schicht aus einem Werkstoff mit geringerer elektrischer Leitfähigkeit als der Werkstoff der Teilchen versehen sind.

2. Elektrode nach Anspruch 1, dadurch gekennzeichnet, daß das auf den Teil der Oberfläche des Sinterkörpers aufgebrachte, gegenüber der Körperflüssigkeit inerte Metall (2) aus Platin, Iridium oder Platin-Iridium-Legierung mit einem Iridium-Gehalt bis zu 20 Gew.-% besteht.

3. Elektrode nach den Ansprüchen 1 und/oder 2, dadurch gekennzeichnet, daß die Teilchen aus Tantal bestehen.

4. Elektrode nach den Ansprüchen 1 bis 3, dadurch gekennzeichnet, daß die Teilchen aus Tantal und das auf einen Teil der Oberfläche des Sinterkörpers aufgebrachte, gegenüber der Körperflüssigkeit inerte Metall aus Platin besteht.

5. Elektrode nach Anspruch 4, dadurch gekennzeichnet, daß das Tantal anodisiert ist.

6. Elektrode nach einem oder mehreren der Ansprüche 1 bis 5, dadurch gekennzeichnet, daß der größte Teil der Poren zum Körpergewebe hin offen ist und einen Durchmesser von 10 bis 50 μm hat.

7. Elektrode nach einem oder mehreren der Ansprüche 1 bis 6, dadurch gekennzeichnet, daß sie becherförmig ausgebildet ist.

8. Elektrode nach Anspruch 7, dadurch gekennzeichnet, daß wenigstens die Becherinnenwand mit dem elektrisch gut leitenden, gegenüber der Körperflüssigkeit inerten Metall versehen ist.

9. Elektrode nach den Ansprüchen 6 und 7, dadurch gekennzeichnet, daß der Becherrand (5) mit dem elektrisch gut leitenden, gegenüber der Körperflüssigkeit inerten Metall versehen ist.

10. Elektrode nach einem oder mehreren der Ansprüche 7 bis 9, dadurch gekennzeichnet, daß die Becherwand Durchgänge (6) aufweist.

11. Elektrode nach Anspruch 10, dadurch gekennzeichnet, daß die Wände (7) der Durchgänge mit dem elektrisch gut leitenden, gegenüber der Körperflüssigkeit inerten Metall versehen sind.

12. Elektrode in Becherform nach einem oder mehreren der vorhergehenden Ansprüche, dadurch gekennzeichnet, daß sie am Becherboden (9) einen Anschlußstutzen (8) für die elektrische Stromzuführung aufweist, dessen Innenwand (10) elektrisch leitend ausgebildet ist.

**Claims**

1. A porous electrode (1) for implantation in organic tissue, more particularly a pacemaker electrode, made from a sintered member (3) of electrically conductive particles which consist of a metal, a metal alloy, or metal compound of an element of the group comprising tantalum, titanium, niobium and zirconium, or of a cobalt-chromium-based alloy, while at least in a part of the surface of the electrode (1) the particles forming this surface zone are provided, at those places which are not interconnected, with a metal which is a good electrical conductor and which is inert to body fluid, while outside the said surface zone of the electrode (1) the particles — at least in a zone adjoining the surface — are provided, at those places which are not interconnected, with a thin coating of a material having a lower electrical conductivity than the material of the particles.

2. An electrode according to claim 1, characterised in that the metal (2) applied to the part of the surface of the sintered member and inert to body fluid consists of platinum, iridium or platinum-iridium alloy having an iridium content of up to 20% by weight.

3. An electrode according to claims 1 and/or 2, characterised in that the particles consist of tantalum.

4. An electrode according to claims 1 to 3, characterised in that the particles consist of tantalum and the metal applied to part of the surface of the sintered member and inert to body fluid consists of platinum.

5. An electrode according to claim 4, characterised in that the tantalum is anodized.

6. An electrode according to one or more of claims 1 to 5, characterised in that most of the pores are open to the body tissue and have a diameter in the range from 10 to 50 μm.

7. An electrode according to one or more of claims 1 to 6, characterised in that it is cup-shaped.

8. An electrode according to claim 7, characterised in that at least the inner wall of the cup is provided with a metal which is a good electrical conductor and which is inert to body fluid.

9. An electrode according to claims 6 and 7, characterised in that the edge (5) of the cup is provided with the metal which is a good electrical conductor and is inert to body fluid.

10. An electrode according to one or more of claims 7 to 9, characterised in that the cup wall has passages (6).

11. An electrode according to claim 10, characterised in that the walls (7) of the passages are provided with the metal which is a good electrical conductor and wich is inert to body fluid.

12. A cup-shaped electrode according to one or more of the preceding claims, characterised in that it has at the base (9) of the cup a connection (8) for the electrical power supply, the inner wall (10) of said connection being made electrically conductive.

**Revendications**

1. Electrode poreuse (1) destinée à être implantée dans un tissu organique, notamment électrode de stimulateur cardiaque, constituée par un corps fritté (3) formé de particules électriquement conductrices qui sont constituées en un métal, en un alliage métallique ou en un composé métallique d'un élément appartenant au groupe du tantale, du titane, du niobium et du zirconium, ou en un alliage à base de cobalt et de chrome, et dans laquelle au moins dans une partie de la surface de l'électrode (1), les particules formant cette région de surface sont munies, aux emplacements non reliés entre eux, d'un métal électriquement bon conducteur et inerte par rapport au liquide du corps, et, à l'extérieur de ladite région de surface de l'électrode (1), les particules sont munies — au moins dans une région jouxtant la surface — d'une couche mince constituée en un matériau possédant une conductibilité électrique plus faible que le matériau des particules, aux emplacements non reliés entre eux.

2. Electrode selon la revendication 1, caractérisée en ce que le métal (2), qui est déposé sur la partie de la surface du corps fritté et qui est inerte par rapport au liquide du corps, est constitué par du platine, de l'iridium ou un alliage de platine ou d'iridium possédant une teneur en iridium allant jusqu'à 20% en poids.

3. Electrode selon les revendications 1 et/ou 2, caractérisée en ce que les particules sont constituées par du tantale.

4. Electrode selon les revendications 1 à 3, caractérisée en ce que les particules sont constituées par du tantale et que le métal, qui est déposé sur une partie de la surface du corps fritté, et est inerte par rapport au liquide du corps, est du platine.

5. Electrode selon la revendication 4, caractérisée en ce que le tantale est anodisé.

6. Electrode selon une ou plusieurs des revendications 1 à 5, caractérisée en ce que la majeure partie des pores est ouverte en direction du tissu du corps et possède un diamètre compris entre 10 et 50 $\mu$m.

7. Electrode selon une ou plusieurs des revendications 1 à 6, caractérisée en ce qu'elle est réalisée sous la forme d'un pot.

8. Electrode selon la revendication 7, caractérisée en ce qu'au moins la paroi intérieure du pot est munie du métal électriquement bon conducteur et inerte vis-à-vis du liquide du corps.

9. Electrode selon les revendications 6 et 7, caractérisée en ce que le bord (5) du pot est muni du métal électriquement bon conducteur, inerte par rapport au liquide du corps.

10. Electrode selon une ou plusieurs des revendications 7 à 9, caractérisée en ce que la paroi du pot possède des passages (6).

11. Electrode selon la revendication 10 caractérisée en ce que les parois (7) des passages sont munies du métal électriquement bon conducteur et inerte vis-à-vis du liquide du corps.

12. Electrode en forme de pot selon une ou plusieurs des revendications précédentes, caractérisée en ce qu'elle comporte, sur le fond (9) du pot, une tubulure de raccordement (8) servant à l'amenée du courant électrique et dont la paroi intérieure (10) est réalisée de manière à être électriquement conductrice.

Fig.1

Fig.2

Fig.3